# EUROPÄISCHE PATENTSCHRIFT

(11) **EP 3 898 619 B1**
(45) Veröffentlichungstag und Bekanntmachung des Hinweises auf die Patenterteilung: **08.02.2023**
(21) Anmeldenummer: 19824281.0
(22) Anmeldetag: 12.12.2019
(51) Int. Cl.: C07D 413/00, C08G 18/48, C08G 18/76, C08G 18/12, C08G 18/28

(54) **FEUCHTIGKEITSHÄRTENDE POLYURETHAN-ZUSAMMENSETZUNG ENTHALTEND OXAZOLIDIN**
MOISTURE-CURABLE POLYURETHANE COMPOSITION CONTAINING OXAZOLIDINE
COMPOSITION DE POLYURÉTHANE DURCISSANT À L'HUMIDITÉ CONTENANT DE L'OXAZOLIDINE

(30) Priorität: 17.12.2018 EP 18213019
(43) Veröffentlichungstag der Anmeldung: 27.10.2021
(73) Patentinhaber: Sika Technology AG, 6340 Baar (CH)
(72) Erfinder: KRAMER, Andreas, 8008 Zürich (CH); BURCKHARDT, Urs, 8049 Zürich (CH)
(74) Vertreter: Sika Patent Attorneys
(86) Internationale Anmeldenummer: PCT/EP2019/084940
(87) Internationale Veröffentlichungsnummer: WO 2020/126841

(56) Entgegenhaltungen:
- JP-A- 2010 024 285

## Beschreibung

### Technisches Gebiet

Die Erfindung betrifft feuchtigkeitshärtende Polyurethan-Zusammensetzungen und ihre Anwendung als elastischer Klebstoff, Dichtstoff oder Beschichtung.

### Stand der Technik

Polyurethan-Zusammensetzungen, welche durch Reaktion von Isocyanatgruppen mit Feuchtigkeit bzw. Wasser vernetzen und dabei zu Elastomeren aushärten, werden insbesondere als Klebstoffe, Dichtstoffe und Beschichtungen in der Bau- und Fertigungsindustrie eingesetzt, beispielsweise zur Bauteilverklebung in der Montage, zur Parkettverklebung, zur Verfüllung von Fugen oder zur Dachabdichtung. Aufgrund ihrer guten Haftung und Elastizität können sie auf die Substrate einwirkende Kräfte, ausgelöst etwa durch Vibrationen oder Temperaturschwankungen, schonend dämpfen. Insbesondere bei dickschichtiger und/oder grossflächiger Anwendung, wie etwa bei Anschlussfugen, der Verklebung grosser Bauteile mit weiten Dimensionstoleranzen oder thermoplastischen Bauteilen, ist es wichtig, dass das Elastomer hoch dehnbar und nicht zu steif ist, d.h. ein niedriges Elastizitätsmodul aufweist, da die auf die Fugenränder bzw. Substrate übertragenen Kräfte sonst zu hoch werden und es zum Bauteilverzug, zu adhäsivem Versagen oder zum Substratbruch kommt, insbesondere bei auf Zug schwachen Substraten wie Beton oder Mörtel. Bei Aussenanwendungen wird diese Problematik noch durch die im Tages- und Jahresverlauf auftretenden Temperaturschwankungen verstärkt, da sich die zu überbrückenden Substratabstände bzw. Fugen in der Kälte ausdehnen und sich das Elastomer beim Abkühlen gleichzeitig versteift.

Für solche Anwendungen sind Elastomere mit weichelastischen Eigenschaften geeignet, welche eine sehr hohe elastische Dehnbarkeit bei niedrigem Elastizitätsmodul aufweisen. Solche Elastomere werden insbesondere mit Zusammensetzungen enthaltend langkettige Polymere und einen insgesamt niedrigen Gehalt an Isocyanatgruppen erhalten. Solche Zusammensetzungen sind aber oft wenig lagerstabil, insbesondere wenn sie viel Füllstoffe und Weichmacher enthalten, welche Feuchtigkeit einschleppen können, und sie zeigen eine ausgeprägte Oberflächenklebrigkeit (Tack), welche lange nach der Applikation anhält und zu Anschmutzungen sowie einer verminderten Witterungsbeständigkeit führt. Auch die zur Reduktion der Blasenbildung bei der Aushärtung oft eingesetzten latenten Härter bringen hier nur beschränkt Abhilfe, da diese typischerweise eine gewisse Steifigkeit bewirken, was für weichelastische Eigenschaften nachteilig ist. Viele dieser Härter vermindern ausserdem die Lagerstabilität zusätzlich, indem sie vorzeitige Vernetzungsreaktionen der Isocyanatgruppen auslösen.

Aus dem Stand der Technik bekannt sind Monooxazolidine abgeleitet von aliphatischen Aldehyden oder Ketonen und ihre Verwendung als Wasserfänger (moisture scavenger) bzw. Trocknungsmittel für Polyurethan-Zusammensetzungen. Kommerziell erhältlich sind beispielsweise 3-Butyl-2-(3-heptyl)-1 ,3-oxazolidin basierend auf N-Butylaminoethanol und 2-Ethylhexanal, zum Beispiel als Incozol 2 (von Incorez), oder 3-Ethyl-2-methyl-2-(3-methylbutyl)-1 ,3-oxazolidin basierend auf N-Ethylaminoethanol und Methylisoamylketon, zum Beispiel als Zoldine^{®} MS-Plus (von Angus Chemical). Solche Trocknungsmittel werden in geringer Menge zum Abfangen von über Füllstoffe und Weichmacher in die Zusammensetzung eingebrachtem Wasser eingesetzt. In vielen Polyurethan-Zusammensetzungen, insbesondere solchen enthaltend sehr reaktive Isocyanatgruppen abgeleitet von aromatischen und/oder sterisch ungehinderten Diisocyanaten, sind sie aber nicht ausreichend lagerstabil.

Weiterhin bekannt sind Bisoxazolidine, welche in einkomponentigen Polyurethanen als latente Härter verwendet werden. Diese sind aber nur eingeschränkt lagerstabil, und sie härten zu eher steifen Elastomeren mit nicht sehr hoher Dehnbarkeit aus.

US 6,255,433 offenbart niedrigmodulige, einkomponentige Dichtstoffe, welche ein Isocyanatgruppen-haltiges Polymer abgeleitet von einer Mischung aus Polyol, Monol und hydroxyfunktionellem Oxazolidin enthalten. Solche Isocyanatgruppen-haltigen Polymere mit angehängten Oxazolidingruppen sind aber hochviskos und nicht sehr lagerstabil. Die damit formulierten Dichtstoffe enthalten erhebliche Mengen an Xylol, das ihre Lagerstabilität verbessert, aber aus Gründen von Geruchs- und VOC-Emissionen unerwünscht ist. Weiterhin wird bei der Polymerherstellung ein Monol mitverwendet. Dies erniedrigt zwar das Elastizitätsmodul, führt aber zu sehr klebrigen Oberflächen.

### Darstellung der Erfindung

Aufgabe der vorliegenden Erfindung ist es daher, eine feuchtigkeitshärtende Polyurethan-Zusammensetzung zur Verfügung zu stellen, welche eine ausgezeichnete Lagerstabilität aufweist und schnell und weitgehend blasenfrei zu einem Elastomer mit geringer Oberflächenklebrigkeit, sehr hoher Dehnbarkeit und sehr niedrigem Elastizitätsmodul aushärtet.

Überraschenderweise wurde gefunden, dass eine Polyurethan-Zusammensetzung wie in Anspruch 1 beschrieben diese Aufgabe löst. Sie enthält ein Isocyanatgruppen-haltiges Polymer und ein Oxazolidin der Formel (I), welches ein Monooxazolidin ist. Das Oxazolidin der Formel (I) ermöglicht eine sehr gute Lagerstabilität, auch bei sehr reaktiven Diisocyanaten, und bei genügender Einsatzmenge eine weitgehend blasenfreie Aushärtung, wobei überraschenderweise ein ausgeprägt weichelastisches Material mit nahezu klebfreier Oberfläche entsteht. Der aus dem Oxazolidin der Formel (I) freigesetzte N-substituierte Aminoalkohol ist gegenüber Isocyanatgruppen difunktionell und bildet ein wenig stark segregierendes Hartsegment, weshalb man beim Einsatz von höheren Oxazolidinmengen ein eher plastisches und klebriges Material erwarten würde. Weiterhin überraschend ist die gute Lagerstabilität zusammen mit sehr reaktiven Isocyanatgruppen, werden Oxazolidine aufgrund mangelnder Lagerstabilität doch sonst vorwiegend in langsam aushärtenden Zusammensetzungen basierend insbesondere auf Isophorondiisocyanat eingesetzt. Besonders überraschend ist die gute Lagerstabilität in Zusammensetzungen basierend auf 4,4'-Diphenylmethandiisocyanat, wo die aus dem Stand der Technik bekannten Oxazolidine nicht oder nur sehr eingeschränkt lagerstabil sind.

Die erfindungsgemässe Polyurethan-Zusammensetzung ermöglicht elastische Klebstoffe, Dichtstoffe oder Beschichtungen mit ausgeprägt weichelastischen Eigenschaften, welche gut lagerstabil sind, schnell aushärten und schon nach kurzer Zeit klebfrei werden, d.h. ihre Oberflächenklebrigkeit (tack) verlieren. Dadurch sind sie gegen Anschmutzen durch beispielsweise Baustaub, Pollen oder Insekten und vor Witterungseinflüssen schon nach kurzer Zeit geschützt und somit ästhetisch ansprechend und langlebig. Aufgrund ihres niedrigen Elastizitätsmoduls bei hoher elastischer Dehnbarkeit sind sie in der Lage, auch grosse Kräfte zu dämpfen und somit schonend auf die Substrate zu übertragen, wodurch es nicht zu Bauteilverzug, Substratbruch oder adhäsivem Versagen kommt. Solche Produkte sind vorteilhaft verwendbar für dickschichtige oder grossflächige Anwendungen, insbesondere für Fugen oder Verklebungen, wo hohe Toleranzen überbrückt werden müssen.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist eine Polyurethan-Zusammensetzung enthaltend mindestens ein Isocyanatgruppen-haltiges Polymer, erhalten aus der Umsetzung von mindestens einem Polyol und mindestens einem Diisocyanat, und mindestens ein Oxazolidin der Formel (I), 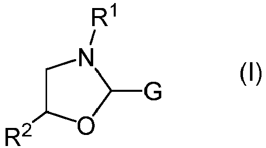 wobei
R¹ für einen Alkyl- oder Cycloalkyl- oder Arylalkyl-Rest mit 1 bis 8 C-Atomen steht, R² für H oder Methyl steht, und
G für einen aromatischen Rest ausgewählt aus der Gruppe bestehend aus Furanyl, Thiophenyl, Pyridinyl, Indenyl, Indanyl, Benzodioxolyl, Benzodioxanyl, Naphthyl, Tetrahydronaphthyl, Anthracenyl und einem Rest der Formel 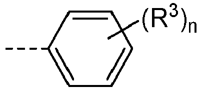 steht, wobei
R³ für Fluoro, Chloro, Bromo, Nitro, Cyano, Phenyl, Phenoxy, (C₁ bis C₅)-Alkyl oder (C₁ bis C₅)-Alkoxy steht, und
n für 0, 1, 2 oder 3 steht.

Für den Fall, dass n für 2 oder 3 steht, können die Reste R³ für gleiche oder verschiedene Reste stehen.

Als "aromatisch" wird ein Rest bezeichnet, welcher über ein aromatisches C-Atom an das jeweilige Molekül gebunden ist.

Als "NCO-Gehalt" wird der Gehalt an Isocyanatgruppen in Gewichts-% bezeichnet. Als "aromatisch" wird eine Isocyanatgruppe bezeichnet, welche direkt an ein aromatisches C-Atom gebunden ist. Isocyanate mit aromatischen Isocyanatgruppen werden entsprechend als "aromatische Isocyanate" bezeichnet.

Als "aliphatisch" wird eine Isocyanatgruppe bezeichnet, welche direkt an ein aliphatisches oder cycloaliphatisches C-Atom gebunden ist. Isocyanate mit ausschliesslich aliphatischen Isocyanatgruppen werden entsprechend als "aliphatische Isocyanate" bezeichnet.

Mit "Poly" beginnende Substanznamen wie Polyol oder Polyisocyanat bezeichnen Substanzen, die formal zwei oder mehr der in ihrem Namen vorkommenden funktionellen Gruppen pro Molekül enthalten.

Als "Molekulargewicht" wird die molare Masse (in Gramm pro Mol) eines Moleküls oder eines Molekül-Rests bezeichnet. Als "mittleres Molekulargewicht" wird das Zahlenmittel des Molekulargewichts (Mₙ) einer polydispersen Mischung von oligomeren oder polymeren Molekülen oder Molekül-Resten bezeichnet. Es wird mittels Gelpermeationschromatographie (GPC) gegen Polystyrol als Standard bestimmt.

Als "lagerstabil" oder "lagerfähig" wird eine Substanz oder eine Zusammensetzung bezeichnet, wenn sie bei Raumtemperatur in einem geeigneten Gebinde während längerer Zeit, typischerweise während mindestens 3 Monaten bis zu 6 Monaten und mehr, aufbewahrt werden kann, ohne dass sie sich in ihren Anwendungs- oder Gebrauchseigenschaften durch die Lagerung in einem für ihren Gebrauch relevanten Ausmass verändert.

Eine gestrichelte Linie in den Formeln in diesem Dokument stellt jeweils die Bindung zwischen einem Substituenten und dem zugehörigen Molekülrest dar. Als "Raumtemperatur" wird eine Temperatur von 23°C bezeichnet.

Bevorzugt hat das Isocyanatgruppen-haltige Polymer einen NCO-Gehalt im Bereich von 0.5 bis 5 Gewichts-%, bevorzugt 0.75 bis 3.2 Gewichts-%, insbesondere 1 bis 2.5 Gewichts-%. Ein solches Polymer ist besonders langkettig und ermöglicht eine hohe Dehnbarkeit des damit erhaltenen Elastomers. Es ist aber aufgrund des geringen NCO-Gehalts auch besonders anspruchsvoll in Bezug auf die Lagerstabilität.

Bevorzugt hat das Isocyanatgruppen-haltige Polymer ein mittleres Molekulargewicht Mₙ im Bereich von 2'000 bis 20'000 g/mol, insbesondere 4'000 bis 15'000 g/mol.

Bevorzugt hat das Isocyanatgruppen-haltige Polymer einen geringen Gehalt an monomerem Diisocyanat, besonders bevorzugt weniger als 2 Gewichts-%, insbesondere weniger als 1 Gewichts-%.

Das Isocyanatgruppen-haltige Polymer wird insbesondere erhalten aus der Umsetzung von mindestens einem Polyol mit einer überstöchiometrischen Menge von mindestens einem Diisocyanat. Die Umsetzung wird bevorzugt unter Feuchtigkeitsausschluss bei einer Temperatur im Bereich von 20 bis 160 °C, insbesondere 40 bis 140 °C, durchgeführt, gegebenenfalls in Anwesenheit geeigneter Katalysatoren.

Das NCO/OH-Verhältnis liegt bevorzugt im Bereich von 1.3/1 bis 10/1. Das nach der Umsetzung der OH-Gruppen im Reaktionsgemisch verbleibende monomere Diisocyanat kann entfernt werden, insbesondere mittels Destillation.

Für den Fall, dass überschüssiges monomeres Diisocyanat mittels Destillation entfernt wird, liegt das NCO/OH-Verhältnis bei der Umsetzung bevorzugt im Bereich von 3/1 bis 7/1, und das erhaltene Isocyanatgruppen-haltige Polymer enthält nach der Destillation bevorzugt höchstens 0.5 Gewichts-%, besonders bevorzugt höchstens 0.3 Gewichts-%, monomeres Diisocyanat. Dabei wird monomeres Diisocyanat insbesondere mittels Kurzwegdestillation im Vakuum entfernt.

Für den Fall, dass kein überschüssiges monomeres Diisocyanat aus dem Polymer entfernt wird, liegt das NCO/OH-Verhältnis bei der Umsetzung bevorzugt im Bereich von 1.3/1 bis 2.5/1.

Gegebenenfalls wird das Polymer unter Mitverwendung von Weichmachern oder Lösemitteln hergestellt, wobei die verwendeten Weichmacher oder Lösemittel keine gegenüber Isocyanaten reaktive Gruppen enthalten.

Bevorzugt ist das Diisocyanat ein handelsübliches aliphatisches, cycloaliphatisches oder aromatisches Diisocyanat, wie insbesondere 1,6-Hexamethylendiisocyanat (HDI), 1-Isocyanato-3,3,5-trimethyl-5-isocyanatomethylcyclohexan (Isophorondiisocyanat oder IPDI), Perhydro-4,4'-diphenylmethandiisocyanat (H₁₂MDI), Cyclohexan-1,3- oder -1,4-diisocyanat, 1,3- oder 1,4-Bis(isocyanatomethyl)cyclohexan, m- oder p-Xylylendiisocyanat (XDI), 2,4-Toluylendiisocyanat oder Gemische davon mit 2,6-Toluylendiisocyanat (TDI), 4,4'-Diphenylmethandiisocyanat, gegebenenfalls mit Anteilen von 2,4'- und/oder 2,2'-Diphenylmethandiisocyanat (MDI), p-Phenylendiisocyanat (PPDI) oder Naphthalin-1,5-diisocyanat (NDI).

Bevorzugt ist das Diisocyanat ausgewählt aus der Gruppe bestehend aus HDI, H₁₂MDI, TDI und MDI. Diese Diisocyanate sind einfach erhältlich und besonders reaktiv. Sie sind aber auch besonders anspruchsvoll in Bezug auf Lagerstabilität.

Davon bevorzugt ist HDI, H₁₂MDI oder MDI. Diese Diisocyanate ermöglichen eine besonders klebfreie Oberfläche.

Besonders bevorzugt als Diisocyanat ist MDI, insbesondere 4,4'-MDI, welches mindestens 97 % des Isomers 4,4'-Diphenylmethandiisocyanat und höchstens 3 % der anderen Isomere enthält. Damit werden Polyurethan-Zusammensetzungen mit besonders schneller Aushärtung erhalten. Damit ist aber auch das Erreichen von guten Lagerstabilitäten und niedrigen Elastizitätsmodulen ganz besonders anspruchsvoll.

Geeignete Polyole sind handelsübliche Polyole oder Mischungen davon, insbesondere
- Polyetherpolyole, insbesondere Polyoxyalkylendiole und/oder Polyoxyalkylentriole, insbesondere Polymerisationsprodukte von Ethylenoxid oder 1,2-Propylenoxid oder 1,2- oder 2,3-Butylenoxid oder Oxetan oder Tetrahydrofuran oder Mischungen davon, wobei diese mit Hilfe eines Startermoleküls mit zwei oder drei aktiven Wasserstoffatomen polymerisiert sein können, insbesondere einem Startermolekül wie Wasser, Ammoniak oder einer Verbindung mit mehreren OH- oder NH-Gruppen wie beispielsweise 1,2-Ethandiol, 1,2- oder 1,3-Propandiol, Neopentylglykol, Diethylenglykol, Triethylenglykol, die isomeren Dipropylenglykole oder Tripropylenglykole, die isomeren Butandiole, Pentandiole, Hexandiole, Heptandiole, Octandiole, Nonandiole, Decandiole, Undecandiole, 1,3- oder 1,4-Cyclohexandimethanol, Bisphenol A, hydriertes Bisphenol A, 1,1,1-Trimethylolethan, 1,1,1-Trimethylolpropan, Glycerin oder Anilin, oder Mischungen der vorgenannten Verbindungen. Ebenfalls geeignet sind Polyetherpolyole mit darin dispergierten Polymerpartikeln, insbesondere solche mit Styrol-Acrylnitril-Partikeln (SAN) oder Polyharnstoff- bzw. Polyhydrazodicarbonamid-Partikeln (PHD).
   Bevorzugte Polyetherpolyole sind Polyoxypropylendiole oder Polyoxypropylentriole, oder sogenannte Ethylenoxid-terminierte (EO-capped bzw. EO-tipped) Polyoxypropylendiole oder -triole. Letztere sind Polyoxyethylen-polyoxypropylen-Mischpolyole, die insbesondere dadurch erhalten werden, dass Polyoxypropylendiole oder -triole nach Abschluss der Polypropoxylierungsreaktion mit Ethylenoxid weiter alkoxyliert werden und dadurch primäre Hydroxylgruppen aufweisen.
   Bevorzugte Polyetherpolyole weisen einen Ungesättigtheitsgrad von weniger als 0.02 mEq/g, insbesondere weniger als 0.01 mEq/g auf.
- Polyesterpolyole, auch Oligoesterole genannt, hergestellt nach bekannten Verfahren, insbesondere der Polykondensation von Hydroxycarbonsäuren bzw. Lactonen oder der Polykondensation von aliphatischen und/oder aromatischen Polycarbonsäuren mit zwei- oder mehrwertigen Alkoholen.
- Polycarbonatpolyole.
- Polyetherpolyesterpolyole.
- Polyacrylat- oder Polymethacrylatpolyole.
- Polyhydroxyfunktionelle Fette oder Öle, beispielsweise natürliche Fette und Öle, inbesondere Ricinusöl; oder durch chemische Modifizierung von natürlichen Fetten und Ölen gewonnene - sogenannte oleochemische - Polyole.
- Polykohlenwasserstoffpolyole, auch Oligohydrocarbonole genannt, wie insbesondere polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen oder Isopren, insbesondere polyhydroxyfunktionelle Acrylonitril/ Butadien-Copolymere, wie sie insbesondere aus Epoxiden oder Aminoalkoholen und carboxylterminierten Acrylonitril/Butadien-Copolymeren (beispielsweise kommerziell erhältlich unter dem Namen Hypro^{®} CTBN oder CTBNX oder ETBN von Emerald Performance Materials) hergestellt werden können; oder hydrierte polyhydroxyfunktionelle Polymere oder Copolymere von Dienen.

Geeignet sind insbesondere auch Mischungen von Polyolen.

Bevorzugt sind Polyetherpolyole, insbesondere Polyoxyalkylendi- oder -triole. Besonders bevorzugt sind Polyoxypropylendi- oder -triole, welche endständig gegebenenfalls Oxyethylengruppen aufweisen.

Bevorzugt sind Polyole mit einem mittleren Molekulargewicht Mₙ im Bereich von 1'000 bis 20'000 g/mol, bevorzugt von 2'000 bis 15'000 g/mol.

Bevorzugt sind Polyole mit einer mittleren OH-Funktionalität im Bereich von 1.6 bis 3.

Die bevorzugten Polyole ermöglichen besonders weiche und beständige Elastomere.

Bei der Herstellung eines Isocyanatgruppen-haltigen Polymers können auch Anteile von zwei- oder mehrfunktionellen Alkoholen mitverwendet werden, insbesondere 1,2-Ethandiol, 1,2-Propandiol, 1,3-Propandiol, 2-Methyl-1,3-propandiol, 1,4-Butandiol, 1,3-Pentandiol, 1,5-Pentandiol, 3-Methyl-1,5-pentandiol, Neopentylglykol, 1,6-Hexandiol, Diethylenglykol, Triethylenglykol, Dipropylenglykol, Tripropylenglykol, 1,3- oder 1,4-Cyclohexandimethanol, ethoxyliertes Bisphenol-A, propoxyliertes Bisphenol-A, Cyclohexandiol, oder alkoxylierte Derivate der genannten Alkohole oder Mischungen der genannten Alkohole.

In einer bevorzugten Ausführungsform enthält das Polyol mindestens ein Triol. Damit wird ein Isocyanatgruppen-haltiges Polymer mit einer besonders hohen NCO-Funktionalität erhalten, was mit dem Oxazolidin der Formel (I) eine besonders klebfreie Oberfläche ermöglicht. Bevorzugt wird als Polyol eine Mischung aus einem oder mehreren Diolen und einem oder mehreren Triolen eingesetzt. Diese Ausführungsform der Erfindung ist insbesondere dann besonders vorteilhaft, wenn die Zusammensetzung keine weiteren reaktiven Bestandteile mit einer Funktionalität von mehr als 2 aufweist, wie beispielsweise Diisocyanat-Oligomere oder Trialdimine.

Die erfindungsgemässe Polyurethan-Zusammensetzung enthält mindestens ein Oxazolidin der Formel (I).

Bevorzugt steht R¹ für Methyl, Ethyl, n- Propyl, n-Butyl, n-Hexyl, Cyclohexyl, 2-Ethylhexyl, oder Benzyl. Diese Oxazolidine sind besonders einfach zugänglich. Besonders bevorzugt steht R¹ für Methyl, Ethyl, n-Butyl oder Benzyl.

Am meisten bevorzugt steht R¹ für Butyl. Damit werden Polyurethan-Zusammensetzungen mit einer besonders attraktiven Kombination aus guter Lagerstabilität, schneller Aushärtung und weichelastischen Eigenschaften erhalten.

Bevorzugt steht R² für H.

Bevorzugt steht G für Furan-2-yl, 1,3-Benzodioxol-5-yl, 1-Naphthyl oder einen Rest der Formel

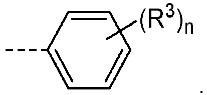

Bevorzugt steht R³ für (C₁ bis C₄)-Alkyl oder (C₁ bis C₄)-Alkoxy, insbesondere für Methyl oder Methoxy.

Besonders bevorzugt steht G für einen Rest der Formel 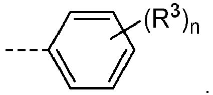 Ein solches Oxazolidin weist die Formel (la) auf, 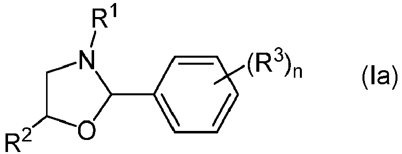 wobei R¹, R², R³ und n die bereits genannten Bedeutungen aufweisen.

Bevorzugt steht n für 0 oder 1 oder 2, insbesondere für 0 oder 1.

Am meisten bevorzugt steht G für Phenyl, 2-Methylphenyl, 3-Methylphenyl, 4-Methylphenyl, 4-Isopropylphenyl oder 4-Methoxyphenyl, insbesondere für Phenyl.

Besonders bevorzugt ist das Oxazolidin der Formel (I) ausgewählt aus der Gruppe bestehend aus

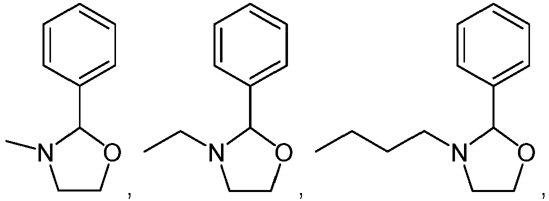

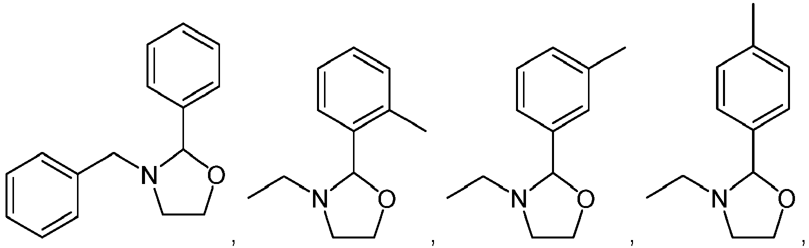

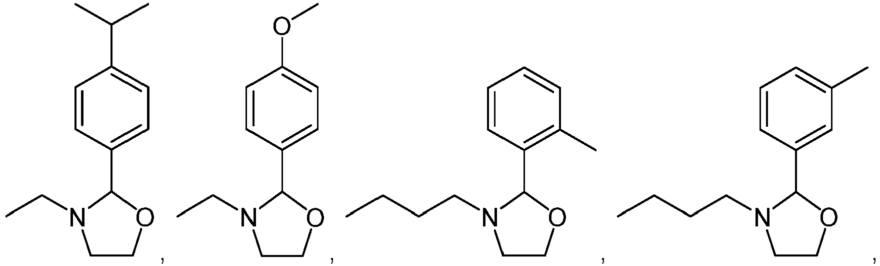

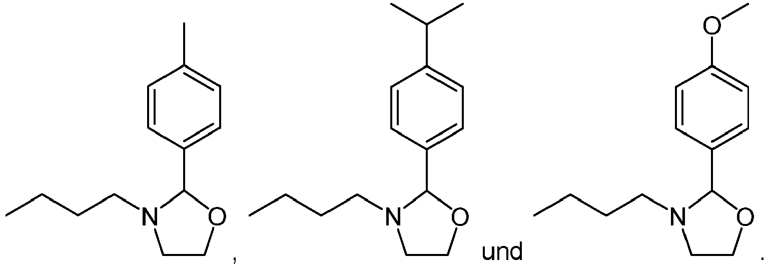

Die bevorzugten Oxazolidine der Formel (I) sind einfach zugänglich, bei Raumtemperatur flüssig und niedrigviskos, zusammen mit reaktiven Isocyanatgruppen gut lagerstabil und ermöglichen eine schnelle Aushärtung.

Das Oxazolidin der Formel (I) wird bevorzugt erhalten aus der Umsetzung von mindestens einem Aminoalkohol der Formel (II) mit mindestens einem Aldehyd der Formel (III) in einer Kondensationsreaktion unter Entfernung von Wasser, 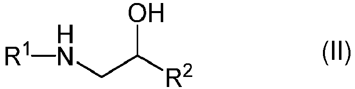 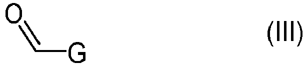 wobei R¹, R², und G die bereits genannten Bedeutungen aufweisen.

Der Aldehyd wird dabei mindestens stöchiometrisch in Bezug auf die NH-Gruppen eingesetzt.

Bevorzugt wird die Umsetzung so geführt, dass der Aminoalkohol der Formel (II) mit dem Aldehyd der Formel (III) zu einer Reaktionsmischung vereint wird und das Kondensationswasser und gegebenenfalls vorhandenes Lösemittel und/oder überschüssiger Aldehyd während oder nach der Vereinigung mit einer geeigneten Methode aus der Reaktionsmischung entfernt werden, gegebenenfalls unter Erwärmen und/oder Anlegen von Vakuum.

Geeignet als Aminoalkohol der Formel (II) ist insbesondere N-Methylethanolamin, N-Ethylethanolamin, N-n-Propylethanolamin, N-Isopropylethanolamin, N-n-Butylethanolamin, N-Isobutylethanolamin, N-2-Butylethanolamin, N-tert.Butylethanolamin, N-n-Hexylethanolamin, N-Isohexylethanolamin, N-(2-Ethylhexyl)-ethanolamin, N-Cyclohexylethanolamin oder N-Benzylethanolamin, insbesondere N-Methylethanolamin, N-Ethylethanolamin, N-n-Butylethanolamin oder N-Benzylethanolamin. Besonders bevorzugt ist N-n-Butylethanolamin.

Geeignet als Aldehyd der Formel (III) ist insbesondere Furan-2-carbaldehyd (Furfural), Furan-3-carbaldehyd, Thiophen-2-carbaldehyd, Thiophen-3-carbaldehyd, Pyridin-2-carbaldehyd, Pyridin-3-carbaldehyd (Nicotinaldehyd), Pyridin-4-carbaldehyd (Isonicotinaldehyd), 1H-Inden-4-carbaldehyd, 1H-Inden-5-carbaldehyd, 1H-Inden-6-carbaldehyd, 1H-Inden-7-carbaldehyd, 4-Indancarbaldehyd, 5-Indancarbaldehyd, 1,3-Benzodioxol-4-carbaldehyd, 1,3-Benzodioxol-5-carbaldehyd, 1,4-Benzodioxan-5-carbaldehyd, 1,4-Benzodioxan-6-carbaldehyd, 1-Naphthaldehyd, 2-Naphthaldehyd, 5,6,7,8-Tetrahydro-1-naphthaldehyd, 5,6,7,8-Tetrahydro-2-naphthaldehyd, 9-Anthracencarbaldehyd, Benzaldehyd, 2-Fluorobenzaldehyd, 3-Fluorobenzaldehyd, 4-Fluorobenzaldehyd, 2-Chlorobenzaldehyd, 3-Chlorobenzaldehyd, 4-Chlorobenzaldehyd, 2,4-Dichlorobenzaldehyd, 3,4-Dichlorobenzaldehyd, 3,5-Dichlorobenzaldehyd, 2-Bromobenzaldehyd, 3-Bromobenzaldehyd, 4-Bromobenzaldehyd, 2-Nitrobenzaldehyd, 3-Nitrobenzaldehyd, 4-Nitrobenzaldehyd, 2,4-Dinitrobenzaldehyd, 3,5-Dinitrobenzaldehyd, 2-Cyanobenzaldehyd, 4-Cyanobenzaldehyd, 4-Phenylbenzaldehyd (Biphenyl-4-carbaldehyd), 3-Phenoxybenzaldehyd, 4-Phenoxybenzaldehyd, 2-Methylbenzaldehyd (o-Tolualdehyd), 3-Methylbenzaldehyd (m-Tolualdehyd), 4-Methylbenzaldehyd (p-Tolualdehyd), 2,4-Dimethylbenzaldehyd, 2,5-Dimethylbenzaldehyd, 2,4,6-Trimethylbenzaldehyd (Mesitylaldehyd), 4-Ethylbenzaldehyd, 4-Isopropylbenzaldehyd (Cuminaldehyd), 4-tert.Butylbenzaldehyd, 2-Methoxybenzaldehyd (o-Anisaldehyd), 3-Methoxybenzaldehyd (m-Anisaldehyd), 4-Methoxybenzaldehyd (Anisaldehyd), 2,3-Dimethoxybenzaldehyd, 2,4-Dimethoxybenzaldehyd, 2,5-Dimethoxybenzaldehyd, 3,4-Dimethoxybenzaldehyd (Veratrumaldehyd), 3,5-Dimethoxybenzaldehyd, 2,4,6-Trimethylbenzaldehyd, 2,4,5-Trimethoxybenzaldehyd (Asaronaldehyd), 2,4,6-Trimethoxybenzaldehyd, 3,4,5-Trimethoxybenzaldehyd, 2-Ethoxybenzaldehyd, 4-Ethoxybenzaldehyd oder 4-Butoxybenzaldehyd. Bevorzugt ist Furan-2-carbaldehyd, 1,3-Benzodioxol-5-carbaldehyd, 1-Naphthaldehyd, Benzaldehyd, 2-Methylbenzaldehyd, 3-Methylbenzaldehyd, 4-Methylbenzaldehyd, 4-Isopropylbenzaldehyd oder 4-Methoxybenzaldehyd. Besonders bevorzugt ist Benzaldehyd.

Das Oxazolidin der Formel (I) setzt bei Hydrolyse den zugrunde liegenden Aminoalkohol der Formel (II) sowie den Aldehyd der Formel (III) frei. Dabei reagiert der Aminoalkohol mit vorhandenen Isocyanatgruppen, wobei die Aminogruppe deutlich schneller als die Hydroxylgruppe und vorhandenes Wasser reagiert.

In der erfindungsgemässen Polyurethan-Zusammensetzung liegt das Zahlenverhältnis zwischen Oxazolidingruppen und Isocyanatgruppen bevorzugt im Bereich von 0.1 bis 0.5.

Besonders bevorzugt liegt das Zahlenverhältnis zwischen Oxazolidingruppen und Isocyanatgruppen im Bereich von 0.2 bis 0.45. In diesem Bereich wirkt das Oxazolidin der Formel (I) nicht nur als Trocknungsmittel, sondern als latenter Härter. Dies ermöglicht eine weitgehend blasenfreie Aushärtung und ausgeprägt weichelastische Eigenschaften.

In einer bevorzugten Ausführungsform der Erfindung enthält die erfindungsgemässe Polyurethan-Zusammensetzung zusätzlich zum Oxazolidin der Formel (I) mindestens ein Aldimin der Formel A-[-N=CHB]_{y}, wobei y für 2 oder 3, A für einen organischen Rest mit 2 bis 25 C-Atomen und B für einen organischen Rest mit 3 bis 30 C-Atomen, insbesondere 4 bis 23 C-Atomen, stehen.

Bevorzugt steht das Aldimin der Formel 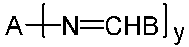 für ein Aldimin der Formel (IV) oder (V), 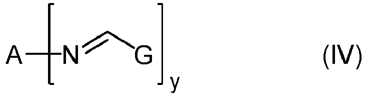 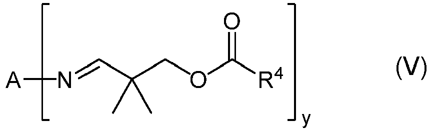 wobei
R⁴ für H oder einen Alkyl-Rest mit 1 bis 17 C-Atomen, insbesondere für einen linearen Alkyl-Rest mit 1 bis 11 C-Atomen, steht,
und G, A und y die bereits beschriebenen Bedeutungen aufweisen.

Bevorzugt steht A für einen gegebenenfalls cyclische Anteile aufweisenden Alkylen-Rest oder einen zwei- oder dreiwertigen Polyoxyalkylen-Rest mit 5 bis 15 C-Atomen, insbesondere für 1,6-Hexylen, (1,5,5-Trimethylcyclohexan-1-yl)methan-1,3 oder α,ω-Polyoxypropylen mit einem mittleren Molekulargewicht im Bereich von 170 bis 300 g/mol oder Trimethylolpropan-gestartetes Tris(ω-polyoxypropylen) mit einem mittleren Molekulargewicht im Bereich von 330 bis 500 g/mol.

Bevorzugt steht G für Phenyl, 2-Methylphenyl, 3-Methylphenyl, 4-Methylphenyl, 4-Isopropylphenyl oder 4-Methoxyphenyl, insbesondere für Phenyl.

Bevorzugt steht R⁴ für Methyl oder Lauryl.

Ein Aldimin der Formel 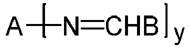 wird insbesondere erhalten durch Umsetzung eines Amins der Formel A-(NH₂)_{y} mit einem Aldehyd der Formel O=CHB unter Entfernung von Kondensationswasser.

Als Amin A-(NH₂)_{y} bevorzugt sind aliphatische oder cycloaliphatische primäre Di- oder Triamine, insbesondere 1,6-Hexamethylendiamin, Isophorondiamin, α,ω-Polyoxypropylendiamine mit einem mittleren Molekulargewicht im Bereich von 200 bis 350 g/mol, insbesondere Jeffamine^{®} D-230 (von Huntsman Corp.), oder Trimethylolpropan-gestartetes Tris(ω-polyoxypropylenamin), insbesondere Jeffamine^{®} T-403 (von Huntsman Corp.).

Als Aldehyd der Formel O=CHB bevorzugt ist Benzaldehyd, 2-Methylbenzaldehyd, 3-Methylbenzaldehyd, 4-Methylbenzaldehyd, 4-Isopropylbenzaldehyd, 4-Methoxybenzaldehyd oder Aldolester von Carbonsäuren, insbesondere 2,2-Dimethyl-3-acetoxypropanal oder 2,2-Dimethyl-3-lauroxyloxypropanal.

Für den Fall, dass die erfindungsgemässe Polyurethan-Zusammensetzung mindestens ein Aldimin der Formel 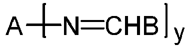 enthält, liegt das Zahlenverhältnis zwischen Oxazolidingruppen und Isocyanatgruppen bevorzugt im Bereich von 0.1 bis 0.3, und das Zahlenverhältnis zwischen der Summe aus Oxazolidin- plus Aldimin-Gruppen und den Isocyanatgruppen liegt bevorzugt im Bereich von 0.3 bis 0.8.

Eine Kombination aus Oxazolidin der Formel (I) und Aldimin ermöglicht sehr lagerstabile weichelastische Zusammensetzungen mit sehr schneller Aushärtung.

Bevorzugt enthält die Polyurethan-Zusammensetzung mindestens einen weiteren Bestandteil, insbesondere ausgewählt aus der Gruppe bestehend aus Füllstoffen, Weichmachern, Diisocyanat-Oligomeren, Katalysatoren und Stabilisatoren.

Geeignete Füllstoffe sind insbesondere gemahlene oder gefällte Calciumcarbonate, welche gegebenenfalls mit Fettsäuren, insbesondere Stearaten, beschichtet sind, Baryte (Schwerspate), Quarzmehle, Quarzsande, Dolomite, Wollastonite, calcinierte Kaoline, Schichtsilikate wie Glimmer oder Talk, Zeolithe, Aluminiumhydroxide, Magnesiumhydroxide, Kieselsäuren inklusive hochdisperse Kieselsäuren aus Pyrolyseprozessen, Zemente, Gipse, Flugaschen, industriell hergestellte Russe, Graphit, Metall-Pulver, beispielsweise von Aluminium, Kupfer, Eisen, Silber oder Stahl, PVC-Pulver oder Hohlkugeln.

Bevorzugt sind Calciumcarbonate, welche gegebenenfalls mit Fettsäuren, insbesondere Stearaten, beschichtet sind, calcinierte Kaoline, hochdisperse Kieselsäuren oder industriell hergestellte Russe.

Geeignete Weichmacher sind insbesondere Carbonsäureester wie Phthalate, insbesondere Diisononylphthalat (DINP), Diisodecylphthalat (DIDP) oder Di(2-propylheptyl)phthalat (DPHP), hydrierte Phthalate bzw. 1,2-Cyclohexandicarbonsäureester, insbesondere hydriertes Diisononylphthalat bzw. Diisononyl-1,2-cyclohexandicarboxylat (DINCH), Terephthalate, insbesondere Bis(2-ethylhexyl)terephthalat (DOTP) oder Diisononylterephthalat (DINT), hydrierte Terephthalate bzw. 1,4-Cyclohexandicarbonsäureester, insbesondere hydriertes Bis(2-ethylhexyl)terephthalat bzw. Bis(2-ethylhexyl)-1,4-cyclohexandicarboxylat oder hydriertes Diisononylterephthalat bzw. Diisononyl-1,4-cyclohexandicarboxylat, Isophthalate, Trimellitate, Adipate, insbesondere Dioctyladipat, Azelate, Sebacate, Benzoate, Glykolether, Glykolester, Weichmacher mit Polyetherstruktur, insbesondere Polypropylenoxid-monole, -diole oder -triole mit blockierten Hydroxylgruppen, insbesondere in der Form von Acetatgruppen, organische Phosphor- oder Sulfonsäureester, Polybutene, Polyisobutene oder von natürlichen Fetten oder Ölen abgeleitete Weichmacher, insbesondere epoxidiertes Soja- oder Leinöl. Bevorzugte Weichmacher sind Phthalate oder Weichmacher mit Polyetherstruktur.

Geeignete Diisocyanat-Oligomere sind insbesondere HDI-Biurete wie Desmodur^{®} N 100 oder N 3200 (von Covestro AG), Tolonate^{®} HDB oder HDB-LV (von Vencorex) oder Duranate^{®} 24A-100 (von Asahi Kasei); HDI-Isocyanurate wie Desmodur^{®} N 3300, N 3600 oder N 3790 BA (alle von Covestro), Tolonate^{®} HDT, HDT-LV oder HDT-LV2 (von Vencorex), Duranate^{®} TPA-100 oder THA-100 (von Asahi Kasei) oder Coronate^{®} HX (vonTosoh Corp.); HDI-Uretdione wie Desmodur^{®} N 3400 (von Covestro); HDI-Iminooxadiazindione wie Desmodur^{®} XP 2410 (von Covestro); HDI-Allophanate wie Desmodur^{®} VP LS 2102 (von Covestro); IPDI-Isocyanurate wie beispielsweise in Lösung als Desmodur^{®} Z 4470 (von Covestro) oder in fester Form als Vestanat^{®} T1890/ 100 (von Evonik Industries); TDI-Oligomere wie Desmodur^{®} IL (von Covestro); oder gemischte Isocyanurate auf Basis TDI/HDI wie Desmodur^{®} HL (von Covestro).

Geeignete Katalysatoren für die Beschleunigung der Reaktion von Isocyanatgruppen sind insbesondere Organozinn(IV)-Verbindungen wie insbesondere Dibutylzinndiacetat, Dibutylzinndilaurat, Dibutylzinndichlorid, Dibutylzinndiacetylacetonat, Dimethylzinndilaurat, Dioctylzinndiacetat, Dioctylzinndilaurat oder Dioctylzinndiacetylacetonat, Komplexverbindungen von Bismut(III) oder Zirkonium(IV), insbesondere mit Liganden ausgewählt aus Alkoholaten, Carboxylaten, 1,3-Diketonaten, Oxinat, 1,3-Ketoesteraten und 1,3-Ketoamidaten, oder tertiäre Aminogruppen enthaltende Verbindungen wie insbesondere 2,2'-Dimorpholinodiethylether (DMDEE).

Geeignete Katalysatoren für die Beschleunigung der Hydrolyse des Oxazolidins oder gegebenenfalls vorhandenen weiteren latenten Härtern sind insbesondere organische Säuren, insbesondere Carbonsäuren wie 2-Ethylhexansäure, Laurinsäure, Stearinsäure, Isostearinsäure, Ölsäure, Neodecansäure, Benzoesäure, Salicylsäure oder 2-Nitrobenzoesäure, organische Carbonsäureanhydride wie Phthalsäureanhydrid, Hexahydrophthalsäureanhydrid oder Hexahydromethylphthalsäureanhydrid, Silylester von Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester. Besonders bevorzugt sind Carbonsäuren, insbesondere aromatische Carbonsäuren wie Benzoesäure, 2-Nitrobenzoesäure oder insbesondere Salicylsäure.

Geeignet sind insbesondere auch Kombinationen von verschiedenen Katalysatoren.

Geeignete Stabilisatoren sind insbesondere Stabilisatoren gegen Oxidation, Wärme, Licht oder UV-Strahlung, insbesondere Titandioxide, Eisenoxide, Zinkoxide, Benzophenone, Benzotriazole, Verbindungen mit 2,6-Di-tert.butylphenol-Gruppen, wie sie beispielsweise unter dem Handelsnamen Irganox^{®} (von BASF) bekannt sind, Verbindungen mit 2,2,6,6-Tetramethylpiperidin-Gruppen, sogenannte HALS (hindered amine light stabilizers), wie sie beispielsweise unter dem Handelsnamen Tinuvin^{®} (von BASF) bekannt sind, oder Phosphor-haltige Verbindungen, wie sie beispielsweise unter dem Handelsnamen Irgafos^{®} (von BASF) bekannt sind.

Die Polyurethan-Zusammensetzung kann weitere Zusätze enthalten, insbesondere
- anorganische oder organische Pigmente, insbesondere Titandioxid, Chromoxide oder Eisenoxide;
- Fasern, insbesondere Glasfasern, Kohlefasern, Metallfasern, Keramikfasern, Kunststofffasern wie Polyamidfasern oder Polyethylenfasern, oder Naturfasern wie Wolle, Cellulose, Hanf oder Sisal;
- Nanofüllstoffe wie Graphen oder Carbon Nanotubes;
- Farbstoffe;
- weitere latente Härter, insbesondere weitere Oxazolidine, Aldimine, Ketimine oder Enamine;
- weitere Isocyanate, insbesondere eine bei Raumtemperatur flüssige Form von MDI, insbesondere ein durch Carbodiimidisierung oder Uretoniminbildung oder Adduktbildung mit Polyolen verflüssigtes 4,4'-MDI, oder ein Gemisch von MDI-Monomeren mit MDI-Homologen (polymeres MDI oder PMDI);
- weitere Trocknungsmittel, insbesondere Molekularsiebpulver, Calciumoxid, hochreaktive Isocyanate wie p-Tosylisocyanat oder Orthoameisensäureester;
- Haftvermittler, insbesondere Organoalkoxysilane, insbesondere Epoxysilane wie insbesondere 3-Glycidoxypropyltrimethoxysilan oder 3-Glycidoxypropyltriethoxysilan, (Meth)acrylosilane, Anhydridosilane, Carbamatosilane, Alkylsilane oder Iminosilane, oder oligomere Formen dieser Silane, oder Titanate;
- weitere Katalysatoren, welche die Reaktion der Isocyanatgruppen beschleunigen;
- Rheologie-Modifizierer, insbesondere Verdickungsmittel, insbesondere Schichtsilikate wie Bentonite, Derivate von Rizinusöl, hydriertes Rizinusöl, Polyamide, Polyamidwachse, Polyurethane, Harnstoffverbindungen, pyrogene Kieselsäuren, Celluloseether oder hydrophob modifizierte Polyoxyethylene;
- Lösemittel, insbesondere Aceton, Methylacetat, tert.Butylacetat, 1-Methoxy-2-propylacetat, Ethyl-3-ethoxypropionat, Diisopropylether, Diethylenglykoldi-ethylether, Ethylenglykoldiethylether, Ethylenglykolmonobutylether, Ethylen-glykolmono-2-ethylhexylether, Acetale wie Propylal, Butylal, 2-Ethylhexylal, Dioxolan, Glycerolformal oder 2,5,7,10-Tetraoxaundecan (TOU), Toluol, Xylol, Heptan, Octan, Naphtha, White Spirit, Petrolether oder Benzin, insbesondere Solvesso^{™}-Typen (von ExxonMobil Chemical Co.), sowie Propylencarbonat, Dimethylcarbonat, Butyrolacton, N-Methylpyrrolidon, N-Ethylpyrrolidon, p-Chlorobenzotrifluorid oder Benzotrifluorid;
- natürliche Harze, Fette oder Öle wie Kolophonium, Schellack, Leinöl, Ricinusöl oder Sojaöl;
- nicht-reaktive Polymere, insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid oder Magnesiumhydroxid, sowie insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)phosphat, isopropyliertes Triphenylphosphat, Mono-, Bis- oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- Additive, insbesondere Netzmittel, Verlaufmittel, Entschäumer, Entlüfter, weitere Stabilisatoren oder Biozide;
oder weitere üblicherweise in feuchtigkeitshärtenden Polyurethan-Zusammensetzungen eingesetzte Substanzen.

Es kann sinnvoll sein, gewisse Substanzen vor dem Einmischen in die Zusammensetzung chemisch oder physikalisch zu trocknen.

Bevorzugt enthält die erfindungsgemässe Polyurethan-Zusammensetzung wenig Lösemittel. Insbesondere enthält sie weniger als 10 Gewichts-%, bevorzugt weniger als 5 Gewichts-%, besonders bevorzugt weniger als 2.5 Gewichts-%, Lösemittel.

Bevorzugt enthält die erfindungsgemässe Polyurethan-Zusammensetzung
- 10 bis 50 Gewichts-% Isocyanatgruppen-haltiges Polymer mit einem NCO-Gehalt im Bereich von 0.5 bis 5 Gewichts-%, erhalten aus der Umsetzung von mindestens einem Polyol und mindestens einem Diisocyanat ausgewählt aus der Gruppe bestehend aus HDI, H₁₂MDI, TDI und MDI,
- 0.1 bis 5 Gewichts-% Oxazolidin der Formel (I),
- 20 bis 60 Gewichts-% Füllstoffe,
- 10 bis 40 Gewichts-% Weichmacher,
und gegebenenfalls weitere Bestandteile, insbesondere Aldimine, Diisocyanat-Oligomere, Katalysatoren oder Stabilisatoren.

Die Polyurethan-Zusammensetzung wird insbesondere unter Ausschluss von Feuchtigkeit hergestellt und bei Umgebungstemperatur in feuchtigkeitsdichten Gebinden aufbewahrt. Ein geeignetes feuchtigkeitsdichtes Gebinde besteht insbesondere aus einem gegebenenfalls beschichteten Metall und/oder Kunststoff und ist insbesondere ein Fass, ein Container, ein Hobbock, ein Eimer, ein Kanister, eine Büchse, ein Beutel, ein Schlauchbeutel, eine Kartusche oder eine Tube.

Die Polyurethan-Zusammensetzung kann in Form einer einkomponentigen oder in Form einer mehrkomponentigen, insbesondere zweikomponentigen, Zusammensetzung vorliegen.

Als "einkomponentig" wird eine Zusammensetzung bezeichnet, bei welcher alle Bestandteile der Zusammensetzung im gleichen Gebinde vorliegen, welche als solche lagerstabil ist und welche mit Feuchtigkeit härtbar ist.

Als "zweikomponentig" wird eine Zusammensetzung bezeichnet, bei welcher die Bestandteile der Zusammensetzung in zwei verschiedenen Komponenten vorliegen, welche in voneinander getrennten Gebinden gelagert und erst kurz vor oder während der Applikation der Zusammensetzung miteinander vermischt werden.

Die Polyurethan-Zusammensetzung ist bevorzugt einkomponentig und feuchtigkeitshärtend. Sie ist bei geeigneter Verpackung und Aufbewahrung lagerstabil, typischerweise während mehreren Monaten bis zu einem Jahr oder länger.

Bei der Aushärtung reagieren die Isocyanatgruppen unter dem Einfluss von Feuchtigkeit mit den aus dem Oxazolidin der Formel (I) freigesetzten Amino- und Hydroxyl-Gruppen und gegebenenfalls vorhandenen weiteren latenten Härtern. Ein Teil der Isocyanatgruppen, insbesondere die gegenüber den freigesetzten Reaktivgruppen überschüssigen, reagieren unter dem Einfluss von Feuchtigkeit untereinander. Die Gesamtheit dieser zur Aushärtung der Zusammensetzung führenden Reaktionen der Isocyanatgruppen wird auch als Vernetzung bezeichnet.

Die zur Aushärtung der Polyurethan-Zusammensetzung benötigte Feuchtigkeit gelangt bevorzugt aus der Luft (Luftfeuchtigkeit) durch Diffusion in die applizierte Zusammensetzung. Dabei bildet sich an den mit Luft in Kontakt stehenden Oberflächen der Zusammensetzung eine feste Schicht ausgehärteter Zusammensetzung (Haut). Die Aushärtung setzt sich entlang der Diffusionsrichtung von aussen nach innen fort, wobei die Haut zunehmend dicker wird und schliesslich die ganze applizierte Zusammensetzung umfasst. Die Feuchtigkeit kann zusätzlich oder vollständig auch aus einem oder mehreren Substrat(en), auf welche(s) die Zusammensetzung appliziert wurde, in die Zusammensetzung gelangen und/oder aus einer Beschleuniger-Komponente stammen, welche der Zusammensetzung bei der Applikation zugemischt oder nach der Applikation mit dieser in Kontakt gebracht wird, beispielsweise durch Bestreichen oder Besprühen.

Die Polyurethan-Zusammensetzung wird bevorzugt bei Umgebungstemperatur appliziert, insbesondere im Bereich von etwa -10 bis 50°C, bevorzugt im Bereich von -5 bis 45°C, insbesondere 0 bis 40°C.

Die Aushärtung der Zusammensetzung erfolgt bevorzugt ebenfalls bei Umgebungstemperatur.

Als Ergebnis der Aushärtung entsteht die ausgehärtete Zusammensetzung.

Die Polyurethan-Zusammensetzung verfügt über eine vergleichsweise lange Offenzeit bei schneller Aushärtung.

Als "Offenzeit" wird die Zeitspanne bezeichnet, während der die Zusammensetzung verarbeitet oder nachbearbeitet werden kann, nachdem der Aushärtungsprozess begonnen hat.

Die Zeit bis zur Ausbildung einer Haut (Hautbildungszeit) stellt dabei ein Mass für die Offenzeit dar.

Bei der Aushärtung wird ein Aldehyd der Formel (III) und gegebenenfalls weitere Aldehyde freigesetzt. Dieser ist, abhängig von seiner Grösse, mehr oder weniger flüchtig und kann aus der ausgehärteten Zusammensetzung verdampfen oder in dieser verbleiben.

Bevorzugt wird die Polyurethan-Zusammensetzung verwendet als Klebstoff oder Dichtstoff oder Beschichtung, wobei der Klebstoff oder Dichtstoff oder die Beschichtung insbesondere weichelastisch ist.

Als Klebstoff und/oder Dichtstoff ist die Zusammensetzung insbesondere geeignet für Klebe- und Abdichtungsanwendungen, insbesondere in der Bau- und Fertigungsindustrie oder im Fahrzeugbau, insbesondere für die Parkettverklebung, Hohlraumversiegelung oder Abdichtung von Fugen, Nähten oder Hohlräumen, insbesondere für Fugen am Bau wie Dilatationsfugen oder Anschlussfugen zwischen Bauteilen. Insbesondere für das Abdichten von Dilatationsfugen an Bauwerken ist ein Dichtstoff mit weichelastischen Eigenschaften besonders geeignet.

Als Beschichtung ist die Zusammensetzung geeignet zum Schutz von Böden oder Mauern, insbesondere als sogenannte Flüssigfolie (liquid applied membrane) zur Abdichtung von Dächern, insbesondere Flachdächern oder schwach geneigten Dachflächen oder Dachgärten, oder im Innern von Gebäuden zur Wasserabdichtung, beispielsweise unter Fliesen oder Keramikplatten in Nasszellen oder Küchen, oder als Nahtabdichtung.

Sie kann auch zu Reparaturzwecken als Abdichtung oder Beschichtung verwendet werden, beispielsweise von undichten Dachmembranen oder sonstigen elastischen Abdichtungen.

Die Polyurethan-Zusammensetzung kann so formuliert sein, dass sie eine pastöse Konsistenz mit hoher Fliessgrenze aufweist, insbesondere für die Verwendung als Klebstoff oder Dichtstoff. Eine solche Zusammensetzung kann aufgespachtelt oder mittels einer geeigneten Vorrichtung unter Druck appliziert werden, beispielsweise mittels einer Tube, Kartuschenpistole oder einer Fasspumpe oder einem Applikationsroboter, wobei die Zusammensetzung gegebenenfalls in Form einer Raupe mit einer im Wesentlichen runden oder dreieckigen Querschnittsfläche ausgetragen wird. Die Schichtdicke der aufgetragenen Zusammensetzung liegt insbesondere im Bereich von 0.5 bis 50 mm, bevorzugt 1 bis 30 mm. Die Polyurethan-Zusammensetzung kann weiterhin so formuliert sein, dass sie flüssig und sogenannt selbstverlaufend oder nur leicht thixotrop ist, insbesondere für die Verwendung als Dichtmasse oder Beschichtung. Eine solche Zusammensetzung kann zur Applikation ausgegossen oder gespachtelt werden. Als Beschichtung kann sie anschliessend flächig bis zur gewünschten Schichtdicke verteilt werden, beispielsweise mittels einer Rolle, Rakel, Zahntraufel oder einem Gummiwischer. In einem Arbeitsgang wird typischerweise eine Schichtdicke im Bereich von 0.5 bis 5 mm, insbesondere 1 bis 3 mm, aufgetragen.

Geeignete Substrate, welche mit der Zusammensetzung verklebt oder abgedichtet oder beschichtet werden können, sind insbesondere
- Glas, Glaskeramik, Beton, Mörtel, Zementestrich, Faserzement, insbesondere Faserzement-Platten, Backstein, Ziegel, Gips, insbesondere Gips-Platten, oder Natursteine wie Granit oder Marmor;
- Reparatur- oder Nivelliermassen auf Basis PCC (Polymer-modifizierter Zementmörtel) oder ECC (Epoxidharz-modifizierter Zementmörtel);
- Metalle oder Legierungen wie Aluminium, Kupfer, Eisen, Stahl, Buntmetalle, inklusive oberflächenveredelte Metalle oder Legierungen wie verzinkte oder verchromte Metalle;
- Asphalt oder Bitumen;
- Leder, Textilien, Papier, Holz, mit Harzen wie Phenol-, Melamin- oder Epoxidharzen gebundene Holzwerkstoffe, Harz-Textil-Verbundwerkstoffe oder weitere sogenannte Polymer-Composites;
- Kunststoffe wie Hart- und Weich-PVC, Polycarbonat, Polystyrol, Polyester, Polyamid, PMMA, ABS, SAN, Epoxidharze, Phenolharze, PUR, POM, TPO, PE, PP, EPM oder EPDM, jeweils unbehandelt oder oberflächenbehandelt, beispielsweise mittels Plasma, Corona oder Flammen;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- Isolierschäume, insbesondere aus EPS, XPS, PUR, PIR, Steinwolle, Glaswolle oder geschäumtem Glas (Foamglas);
- beschichtete oder lackierte Substrate, insbesondere lackierte Fliesen, gestrichener Beton, pulverbeschichtete Metalle oder Legierungen oder lackierte Bleche;
- Farben oder Lacke, insbesondere Automobildecklacke.

Die Substrate können bei Bedarf vor dem Applizieren vorbehandelt werden, insbesondere durch physikalische und/oder chemische Reinigungsverfahren oder das Aufbringen eines Aktivators oder eines Primers.

Verklebt und/oder abgedichtet werden können zwei gleichartige oder zwei verschiedene Substrate.

Ein weiterer Gegenstand der Erfindung ist ein Verfahren zum Verkleben und/oder Abdichten, umfassend die Schritte
(i) Applizieren der beschriebenen Polyurethan-Zusammensetzung
   - auf ein erstes Substrat und Kontaktieren der Zusammensetzung mit mindestens einem zweiten Substrat innerhalb der Offenzeit der Zusammensetzung, oder
   - auf ein erstes und auf ein zweites Substrat und Fügen der beiden Substrate innerhalb der Offenzeit der Zusammensetzung, oder
   - zwischen mindestens zwei Substrate,
(ii) Aushärten der Zusammensetzung durch Kontakt mit Feuchtigkeit.

Ein weiterer Gegenstand der Erfindung ist ein Verfahren zum Beschichten und/oder Abdichten, umfassend die Schritte
(i) Applizieren der beschriebenen Polyurethan-Zusammensetzung auf mindestens ein Substrat,
(ii) Aushärten der Zusammensetzung durch Kontakt mit Feuchtigkeit.

Im Fall einer zwei- oder mehrkomponentigen Zusammensetzung erfolgt vor Schritt (i) jeweils das Vermischen der Komponenten.

Ein weiterer Gegenstand der Erfindung ist ein Artikel, welcher aus dem Verfahren zum Verkleben und/oder Abdichten oder dem Verfahren zum Beschichten und/oder Abdichten erhalten wird. Dieser ist mit der beschriebenen Polyurethan-Zusammensetzung verklebt, abgedichtet oder beschichtet.

Der Artikel ist insbesondere ein Bauwerk des Hoch- oder Tiefbaus oder ein Teil davon, insbesondere eine Brücke, ein Dach, ein Treppenhaus, ein Boden oder eine Fassade, oder ein Anbauteil davon, oder er ist ein industrielles Gut oder ein Konsumgut, insbesondere ein Fenster, eine Haushaltmaschine oder ein Transportmittel, oder ein Anbauteil davon.

Die erfindungsgemässe Polyurethan-Zusammensetzung weist vorteilhafte Eigenschaften auf. Sie ist unter Ausschluss von Feuchtigkeit lagerstabil. Sie verfügt über eine ausreichend lange Offenzeit, um über einen gewissen Zeitraum nach der Applikation nachbearbeitbar zu sein. Die Aushärtung erfolgt schnell, zuverlässig und weitgehend blasenfrei, so dass die Zusammensetzung ohne Einschränkung auch unter klimatisch ungünstigen Bedingungen wie hoher Luftfeuchtigkeit und/oder hoher Temperatur oder unter Einsatz von wasserhaltigen Beschleunigerkomponenten verwendbar ist. Die dabei gebildete gehärtete Oberfläche ist nach kurzer Zeit überraschend klebfrei, was für ein ausgeprägt weichelastisches Material schwierig zu erreichen und insbesondere bei der Anwendung auf Baustellen sehr wertvoll ist. Die Aushärtung führt zu einem weichelastischen Material, welches über eine sehr hohe elastische Dehnbarkeit bei einem niedrigen Elastizitätsmodul verfügt.

Im Folgenden sind Ausführungsbeispiele aufgeführt, welche die beschriebene Erfindung näher erläutern sollen.

Als "Normklima" ("NK") wird eine Temperatur von 23±1°C und eine relative Luftfeuchtigkeit von 50±5% bezeichnet.

Die verwendeten Chemikalien waren, sofern nicht anders bezeichnet, von Sigma-Aldrich Chemie GmbH.

### Herstellung von Oxazolidinen:

Die **Aminzahl** (inklusive blockierte Aminogruppen) wurde bestimmt mittels Titration (mit 0.1_{N} HClO₄ in Essigsäure gegen Kristallviolett).

Die **Viskosität** wurde mit einem thermostatisierten Kegel-Platten-Viskosimeter Rheotec RC30 (Kegeldurchmesser 20 mm, Kegelwinkel 1°, Kegelspitze-Platten-Abstand 0.05 mm, Scherrate 150 s⁻¹ für Viskositäten < 7 mPa s, Scherrate 100 s⁻¹ für Viskositäten von 7 bis 100 mPa·s, Scherrate 10 s⁻¹ für Viskositäten > 0.1 Pa·s) gemessen.

**Infrarotspektren** (FT-IR) wurden als unverdünnte Filme auf einem mit horizontaler ATR-Messeinheit mit Diamant-Kristall ausgestatteten FT-IR Gerät Nicolet iS5 von Thermo Scientific gemessen. Die Absorptionsbanden sind in Wellenzahlen (cm⁻¹) angegeben.

### Oxazolidin Ox-1: 3-Methyl-2-phenyl-1,3-oxazolidin

26.29 g N-Methylethanolamin wurden in einem Rundkolben mit Wasserabscheider (Dean-Stark-Apparatur) vorgelegt, mit 37.88 g Benzaldehyd, 65 ml Cyclohexan und 1.25 g Salicylsäure versetzt und die Reaktionsmischung bei 120°C unter Rückfluss gekocht, bis alles Wasser abgeschieden war. Anschliessend wurde die Reaktionsmischung abgekühlt, filtriert und die flüchtigen Bestandteile unter Vakuum am Rotationsverdampfer bei 80°C entfernt. Es wurde eine gelbliche Flüssigkeit mit einer Viskosität von 5.6 mPa·s bei 20°C, einer Aminzahl von 333 mg KOH/g und einem berechneten Oxazolidin-Equivalentgewicht von 163.2 g/eq erhalten.

FT-IR: 3030, 2974, 2947, 2885, 2843, 2792, 2709, 1702, 1595, 1491, 1456, 1419, 1380, 1339, 1308, 1292, 1249, 1220, 1205, 1160, 1101, 1052, 1036, 1026, 975, 956, 922, 887, 860, 843, 754, 698.

### Oxazolidin Ox-2: 3-Ethyl-2-phenyl-1,3-oxazolidin

31.20 g N-Ethylethanolamin wurden wie für Oxazolidin **Ox-1** beschrieben mit 37.88 g Benzaldehyd umgesetzt. Es wurde eine gelbliche Flüssigkeit mit einer Viskosität von 6.1 mPa·s bei 20°C, einer Aminzahl von 308 mg KOH/g und einem berechneten Oxazolidin-Equivalentgewicht von 177.3 g/eq erhalten.

### Oxazolidin Ox-3: 3-Butyl-2-phenyl-1,3-oxazolidin

41.02 g N-n-Butylethanolamin wurden wie für Oxazolidin **Ox-1** beschrieben mit 37.88 g Benzaldehyd umgesetzt. Es wurde eine gelbliche Flüssigkeit mit einer Viskosität von 8.6 mPa·s bei 20°C, einer Aminzahl von 267 mg KOH/g und einem berechneten Oxazolidin-Equivalentgewicht von 205.3 g/eq erhalten.

FT-IR: 2955, 2931, 2872, 2801, 2714, 1704, 1593, 1492, 1456, 1377, 1342, 1307, 1292, 1248, 1215, 1203, 1178, 1152, 1086, 1063, 956, 924, 912, 844, 756, 736, 695.

### Oxazolidin Ox-4: 3-Benzyl-2-phenyl-1,3-oxazolidin

52.92 g N-Benzylethanolamin wurden wie für Oxazolidin **Ox-1** beschrieben mit 37.88 g Benzaldehyd umgesetzt. Es wurde eine gelbliche Flüssigkeit mit einer Viskosität von 71 mPa·s bei 20°C, einer Aminzahl von 230 mg KOH/g und einem berechneten Oxazolidin-Equivalentgewicht von 239.3 g/eq erhalten.

FT-IR: 3061, 3028, 2942, 2884, 2801, 2713, 1952, 1882, 1811, 1703, 1604, 1586, 1494, 1454, 1385, 1371, 1340, 1307, 1292, 1252, 1215, 1203, 1161, 1127, 1101, 1056, 1025, 1001, 957, 922, 912, 889, 847, 757, 737, 696.

### Oxazolidin Ox-5: 2,3-Diphenyl-1,3-oxazolidin

48.01 g N-Phenylethanolamin (Anilin-ethanol) wurden wie für Oxazolidin **Ox-1** beschrieben mit 37.88 g Benzaldehyd umgesetzt. Es wurde eine gelbliche Flüssigkeit mit einer Aminzahl von 250 mg KOH/g und einem berechneten Oxazolidin-Equivalentgewicht von 225.3 g/eq erhalten, welche bei Raumtemperatur allmählich kristallisierte.

### Oxazolidin Ox-6: Bis-Oxazolidin (1,6-Bis((2-(2-phenyl-1,3-oxazolidin-3-yl)ethoxy)carbonylamino)hexan)

Zuerst wurde 3-(2-Hydroxyethyl)-2-phenyl-1,3-oxazolidin hergestellt, indem 63.09 g Diethanolamin wie für Oxazolidin **Ox-1** beschrieben mit 37.88 g Benzaldehyd umgesetzt wurde. Es wurde eine dunkelgelbe Flüssigkeit mit einer Aminzahl von 288.2 mg KOH/g erhalten.

37.80 g des erhaltenen Produkts wurden unter Stickstoffatmosphäre in einem Rundkolben vorgelegt und aufgewärmt. Bei 80°C wurden tropfenweise 8.33 g 1,6-Hexamethylendiisocyanat zugegeben und dann bei 80°C gerührt, bis mittels IR-Spektroskopie keine Isocyanatgruppen mehr nachweisbar waren. Es wurde ein bei Raumtemperatur festes, gelbes Material mit einer Aminzahl von 181.4 mg KOH/g und einem berechneten Oxazolidin-Equivalentgewicht von 279 g/eq erhalten, welches bei 60°C eine Viskosität von 584 Pa s aufwies.

**Incozol 2** (von Incorez): 3-Butyl-2-(3-heptyl)-1,3-oxazolidin, basierend auf N-Butylethanolamin und 2-Ethylhexanal, mit einem berechneten Oxazolidin-Equivalentgewicht von 227.4 g/eq.

Die Oxazolidine **Ox-1** bis **Ox-4** sind Oxazolidine der Formel (I). Die Oxazolidine **Ox-5**, **Ox-6** und **Incozol 2** entsprechen nicht der Formel (I) und dienen als Vergleich.

### Herstellung von Isocyanatgruppen-haltigen Polymeren:

### Polymer P1:

1300 g Polyoxypropylen-Diol (Acclaim^{®} 4200, von Covestro; OH-Zahl 28.5 mg KOH/g), 2600 g Polyoxypropylen-Triol (Caradol^{®} MD34-02, von Shell; OH-Zahl 35.0 mg KOH/g), 600 g 4,4'-Diphenylmethandiisocyanat (Desmodur^{®} 44 MC L, von Covestro) und 500 g Diisodecylphthalat wurden bei 80 °C zu einem NCOterminierten Polyurethanpolymeren mit einem Gehalt an freien Isocyanat-Gruppen von 2.1 Gewichts-% umgesetzt.

### Herstellung von Polyurethan-Zusammensetzungen:

### Zusammensetzungen Z1 bis Z14:

Für jede Zusammensetzung wurden die in den Tabellen 1 bis 3 angegebenen Inhaltsstoffe in den angegebenen Mengen (in Gewichtsteilen) mittels eines Zentrifugalmischers (SpeedMixer^{™} DAC 150, FlackTek Inc.) unter Feuchtigkeitsausschluss während einer Minute bei 3000 rpm vermischt und unter Feuchtigkeitsausschluss aufbewahrt. Jede Zusammensetzung wurde folgendermassen geprüft:
Als Mass für die Lagerstabilität wurden die **Viskosität** am Folgetag der Herstellung **(1d RT)**, sowie nach einer Lagerung im verschlossenen Gebinde während 7 Tagen bei Raumtemperatur **(7d RT)** und nach einer Lagerung im verschlossenen Gebinde während 7 Tagen in einem 60 °C warmen Umluftofen **(7d 60°C)** bestimmt, wie vorgängig beschrieben.

Als Mass für die Offenzeit wurde die **Hautbildungszeit** bestimmt. Dazu wurden einige Gramm der Zusammensetzung in einer Schichtdicke von ca. 2 mm auf Pappkarton aufgetragen und im Normklima die Zeitdauer bestimmt, bis beim leichten Antippen der Oberfläche der Zusammensetzung mittels einer Pipette aus LDPE erstmals keine Rückstände auf der Pipette mehr zurückblieben.

Zur Bestimmung der mechanischen Eigenschaften wurde jede Zusammensetzung zwischen zwei wachsbeschichteten Transferdruckpapieren zu einem Film von 2 mm Dicke verpresst und während 7 Tagen im Normklima gelagert. Nach Entfernen der Wachspapiere wurden einige Hanteln mit einer Länge von 75 mm bei einer Steglänge von 30 mm und einer Stegbreite von 4 mm aus dem Film ausgestanzt und diese gemäss DIN EN 53504 bei einer Zuggeschwindigkeit von 200 mm/min auf **Zugfestigkeit** (Bruchkraft), **Bruchdehnung**, **E-Modul 25%** (bei 0.5-25% Dehnung) und **E-Modul 100%** (bei 0.5-100% Dehnung) geprüft.

Die **Shore A** Härte wurde bestimmt nach DIN 53505 an während 7 Tagen im Nomklima gehärteten Prüfkörpern.

Der **Aspekt** wurde an den hergestellten Prüfkörpern für die Shore Härte in Bezug auf die Anwesenheit von Blasen und die Oberflächenklebrigkeit durch Antippen mit dem Finger beurteilt. Dabei bedeutet "klebfrei" bzw. "(klebfrei)", dass der Film ganz bzw. nahezu ohne Oberflächenklebrigkeit ist.

Die Resultate sind in den Tabellen 1 bis 3 angegeben.

Bei den mit "(Ref.)" gekennzeichneten Zusammensetzungen handelt es sich um Vergleichsbeispiele.

Das **Verdickungsmittel** wurde vorgängig hergestellt, indem in einem Vakuummischer 300 g Diisodecylphthalat und 48 g 4,4'-Diphenylmethandiisocyanat (Desmodur^{®} 44 MC L, von Covestro) vorgelegt, leicht aufgewärmt und anschliessend unter starkem Rühren 27 g Monobutylamin langsam zugetropft wurde. Die entstehende Paste wurde unter Vakuum und Kühlung eine Stunde weitergerührt.

Das **Aldimin-1** wurde hergestellt, indem 50.00 g 2,2-Dimethyl-3-lauroyloxy-propanal in einem Rundkolben unter Stickstoffatmosphäre vorgelegt, unter Rühren 13.93 g 3-Aminomethyl-3,5,5-trimethylcyclohexylamin (Vestamin^{®} IPD, von Evonik Industries) zugegeben und anschliessend die flüchtigen Bestandteile bei 80 °C und 10 mbar Vakuum entfernt wurden. Es wurde eine blassgelbe, geruchlose Flüssigkeit mit einer Viskosität von 0.2 Pa s bei 20 °C und einer Aminzahl von 153.0 mg KOH/g erhalten.

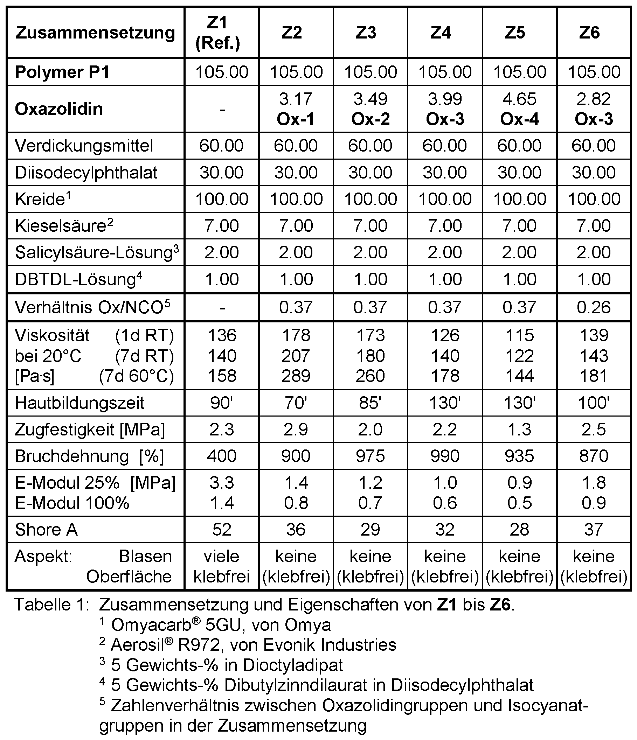

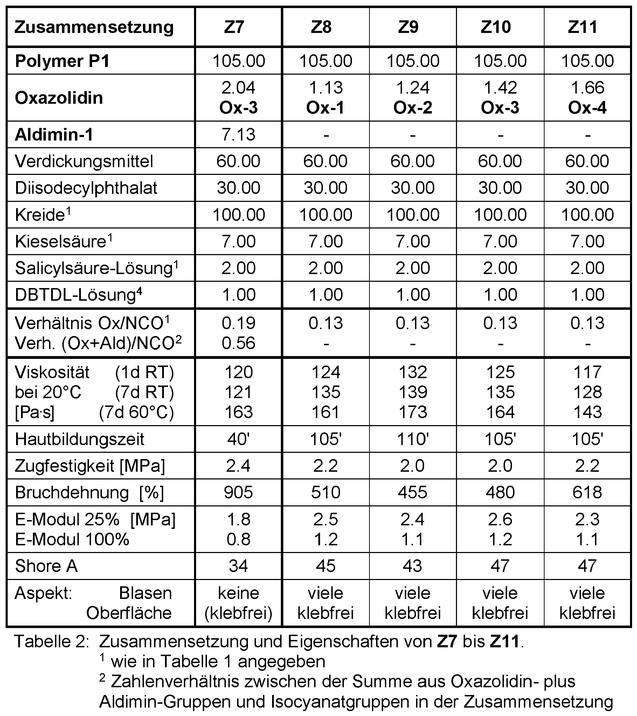

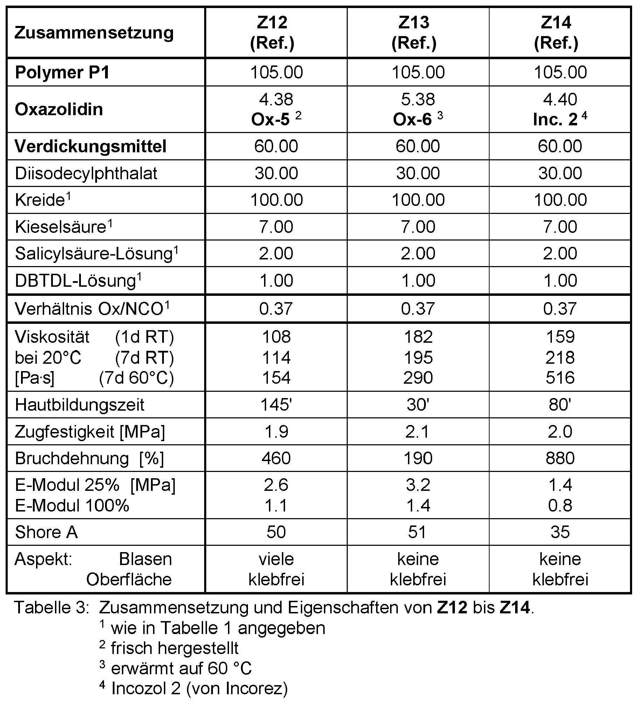

## Patentansprüche

1. Polyurethan-Zusammensetzung enthaltend mindestens ein Isocyanat-gruppen-haltiges Polymer, erhalten aus der Umsetzung von mindestens einem Polyol und mindestens einem Diisocyanat, und mindestens ein Oxazolidin der Formel (I), 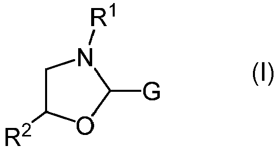 wobei
R¹ für einen Alkyl- oder Cycloalkyl- oder Arylalkyl-Rest mit 1 bis 8 C-Atomen steht,
R² für H oder Methyl steht, und
G für einen aromatischen Rest ausgewählt aus der Gruppe bestehend aus Furanyl, Thiophenyl, Pyridinyl, Indenyl, Indanyl, Benzodioxolyl, Benzodioxanyl, Naphthyl, Tetrahydronaphthyl, Anthracenyl und einem Rest der Formel 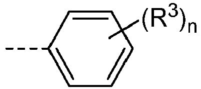 steht, wobei
R³ für Fluoro, Chloro, Bromo, Nitro, Cyano, Phenyl, Phenoxy, (C₁ bis C₅)-Alkyl oder (C₁ bis C₅)-Alkoxy steht, und
n für 0, 1, 2 oder 3 steht.

2. Polyurethan-Zusammensetzung gemäss Anspruch 1, **dadurch gekennzeichnet, dass** das Isocyanatgruppen-haltige Polymer einen NCO-Wert im Bereich von 0.5 bis 5 Gewichts-% aufweist.

3. Polyurethan-Zusammensetzung gemäss einem der Ansprüche 1 oder 2, **dadurch gekennzeichnet, dass** das Diisocyanat ausgewählt ist aus der Gruppe bestehend aus 1,6-Hexamethylendiisocyanat, Perhydro-4,4'-diphenylmethandiisocyanat, 2,4-Toluylendiisocyanat oder Gemische davon mit 2,6-Toluylendiisocyanat, und 4,4'-Diphenylmethandiisocyanat, gegebenenfalls mit Anteilen von 2,4'- und/oder 2,2'-Diphenylmethandiisocyanat.

4. Polyurethan-Zusammensetzung gemäss einem der Ansprüche 1 bis 3, **dadurch gekennzeichnet, dass** das Polyol mindestens ein Triol enthält.

5. Polyurethan-Zusammensetzung gemäss einem der Ansprüche 1 bis 4, **dadurch gekennzeichnet, dass** R¹ für Methyl, Ethyl, n-Butyl oder Benzyl steht.

6. Polyurethan-Zusammensetzung gemäss einem der Ansprüche 1 bis 5, **dadurch gekennzeichnet, dass** G für Furan-2-yl, 1,3-Benzodioxol-5-yl, 1-Naphthyl oder einen Rest der Formel 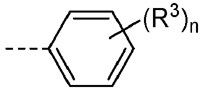 steht.

7. Polyurethan-Zusammensetzung gemäss einem der Ansprüche 1 bis 6, **dadurch gekennzeichnet, dass** G für Phenyl, 2-Methylphenyl, 3-Methylphenyl, 4-Methylphenyl, 4-Isopropylphenyl oder 4-Methoxyphenyl steht.

8. Polyurethan-Zusammensetzung gemäss einem der Ansprüche 1 bis 7, **dadurch gekennzeichnet, dass** das Zahlenverhältnis zwischen Oxazolidingruppen und Isocyanatgruppen im Bereich von 0.2 bis 0.45 liegt.

9. Polyurethan-Zusammensetzung gemäss einem der Ansprüche 1 bis 8, **dadurch gekennzeichnet, dass** zusätzlich mindestens ein Aldimin der Formel 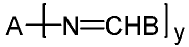 vorhanden ist, wobei y für 2 oder 3, A für einen organischen Rest mit 2 bis 25 C-Atomen und B für einen organischen Rest mit 3 bis 30 C-Atomen stehen.

10. Polyurethan-Zusammensetzung gemäss Anspruch 9, **dadurch gekennzeichnet, dass** das Zahlenverhältnis zwischen Oxazolidingruppen und Isocyanatgruppen im Bereich von 0.1 bis 0.3 und das Zahlenverhältnis zwischen der Summe aus Oxazolidin- plus Aldimin-Gruppen und den Isocyanatgruppen im Bereich von 0.3 bis 0.8 liegen.

11. Polyurethan-Zusammensetzung gemäss einem der Ansprüche 1 bis 10, **dadurch gekennzeichnet, dass** mindestens ein weiterer Bestandteil ausgewählt aus der Gruppe bestehend aus Füllstoffen, Weichmachern, Diisocyanat-Oligomeren, Katalysatoren und Stabilisatoren enthalten ist.

12. Polyurethan-Zusammensetzung gemäss einem der Ansprüche 1 bis 11, **dadurch gekennzeichnet, dass** sie einkomponentig und feuchtigkeitshärtend ist.

13. Verfahren zum Verkleben und/oder Abdichten, umfassend die Schritte
(i) Applizieren der Polyurethan-Zusammensetzung gemäss einem der Ansprüche 1 bis 12
- auf ein erstes Substrat und Kontaktieren der Zusammensetzung mit mindestens einem zweiten Substrat innerhalb der Offenzeit der Zusammensetzung, oder
- auf ein erstes und auf ein zweites Substrat und Fügen der beiden Substrate innerhalb der Offenzeit der Zusammensetzung, oder
- zwischen mindestens zwei Substrate,
(ii) Aushärten der Zusammensetzung durch Kontakt mit Feuchtigkeit.

14. Verfahren zum Beschichten und/oder Abdichten, umfassend die Schritte
(i) Applizieren der Polyurethan-Zusammensetzung gemäss einem der Ansprüche 1 bis 12 auf mindestens ein Substrat,
(ii) Aushärten der Zusammensetzung durch Kontakt mit Feuchtigkeit.

15. Artikel, erhalten aus einem Verfahren gemäss einem der Ansprüche 13 oder 14.

## Claims

1. A polyurethane composition comprising at least one polymer containing isocyanate groups, obtained from the reaction of at least one polyol and at least one diisocyanate, and at least one oxazolidine of the formula (I) 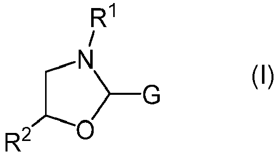 where
R¹ is an alkyl or cycloalkyl or arylalkyl radical having 1 to 8 carbon atoms, R² is H or methyl, and
G is an aromatic radical selected from the group consisting of furanyl, thiophenyl, pyridinyl, indenyl, indanyl, benzodioxolyl, benzodioxanyl, naphthyl, tetrahydronaphthyl, anthracenyl and a radical of the formula 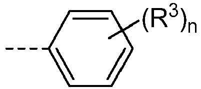 where
R³ is fluoro, chloro, bromo, nitro, cyano, phenyl, phenoxy, (C₁ to Cs)-alkyl or (C₁ to Cs)-alkoxy, and
n is 0, 1, 2 or 3.

2. The polyurethane composition as claimed in claim 1, **characterized in that** the polymer containing isocyanate groups has an NCO value in the range from 0.5% to 5% by weight.

3. The polyurethane composition as claimed in either of claims 1 and 2, **characterized in that** the diisocyanate is selected from the group consisting of hexamethylene 1,6-diisocyanate, perhydro(diphenylmethane 4,4'-diisocyanate), tolylene 2,4-diisocyanate or mixtures thereof with tolylene 2,6-diisocyanate, and diphenylmethane 4,4'-diisocyanate, optionally with fractions of diphenylmethane 2,4'- and/or 2,2'-diisocyanate.

4. The polyurethane composition as claimed in any of claims 1 to 3, **characterized in that** the polyol contains at least one triol.

5. The polyurethane composition as claimed in any of claims 1 to 4, **characterized in that** R¹ is methyl, ethyl, n-butyl or benzyl.

6. The polyurethane composition as claimed in any of claims 1 to 5, **characterized in that** G is furan-2-yl, 1,3-benzodioxol-5-yl, 1-naphthyl or a radical of the formula

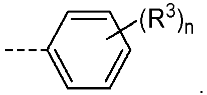

7. The polyurethane composition as claimed in any of claims 1 to 6, **characterized in that** G is phenyl, 2-methylphenyl, 3-methylphenyl, 4-methylphenyl, 4-isopropylphenyl or 4-methoxyphenyl.

8. The polyurethane composition as claimed in any of claims 1 to 7, **characterized in that** the numerical ratio between oxazolidine groups and isocyanate groups is in the range from 0.2 to 0.45.

9. The polyurethane composition as claimed in any of claims 1 to 8, **characterized in that** at least one aldimine of the formula 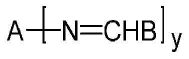 is additionally present, where y is 2 or 3, A is an organic radical having 2 to 25 carbon atoms, and B is an organic radical having 3 to 30 carbon atoms.

10. The polyurethane composition as claimed in claim 9, **characterized in that** the numerical ratio between oxazolidine groups and isocyanate groups is in the range from 0.1 to 0.3, and the numerical ratio between the sum total of oxazolidine plus aldimine groups and the isocyanate groups is in the range from 0.3 to 0.8.

11. The polyurethane composition as claimed in any of claims 1 to 10, **characterized in that** at least one further constituent selected from the group consisting of fillers, plasticizers, diisocyanate oligomers, catalysts and stabilizers is present.

12. The polyurethane composition as claimed in any of claims 1 to 11, **characterized in that** it is a one-component moisture-curing composition.

13. A method of bonding and/or sealing, comprising the steps of
(i) applying the polyurethane composition as claimed in any of claims 1 to 12
- to a first substrate and contacting the composition with at least one second substrate within the open time of the composition, or
- to a first and to a second substrate and joining the two substrates within the open time of the composition, or
- between at least two substrates,
(ii)curing the composition by contact with moisture.

14. A method of coating and/or sealing, comprising the steps of
(i) applying the polyurethane composition as claimed in any of claims 1 to 12 to at least one substrate,
(ii)curing the composition by contact with moisture.

15. An article obtained from a method as claimed in either of claims 13 and 14.

## Revendications

1. Composition de polyuréthane contenant au moins un polymère contenant des groupes isocyanate, obtenu par la transformation d'au moins un polyol et d'au moins un diisocyanate, et au moins une oxazolidine de formule (I), 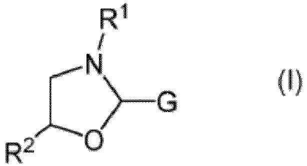
R¹ représentant un radical alkyle ou cycloalkyle ou arylalkyle comportant 1 à 8 atomes de C,
R² représentant H ou méthyle, et
G représentant un radical aromatique choisi dans le groupe constitué par furannyle, thiophényle, pyridinyle, indényle, indanyle, benzodioxolyle, benzodioxanyle, naphtyle, tétrahydronaphtyle, anthracényle et un groupe de formule 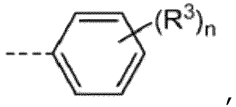
R³ représentant fluoro, chloro, bromo, nitro, cyano, phényle, phénoxy, (C₁-C₅)-alkyle ou (C₁-C₅)-alcoxyle, et
n représentant 0, 1, 2 ou 3.

2. Composition de polyuréthane selon la revendication 1, **caractérisée en ce que** le polymère contenant des groupes isocyanate présente un indice de NCO dans la plage de 0,5 à 5 % en poids.

3. Composition de polyuréthane selon l'une quelconque des revendications 1 et 2, **caractérisée en ce que** le diisocyanate est choisi dans le groupe constitué par le 1,6-diisocyanate de hexaméthylène, le 4,4'-diisocyanate de perhydro-diphénylméthane, le 2,4-diisocyanate de toluylène ou des mélanges de ceux-ci avec le 2,6-diisocyanate de toluylène, et le 4,4'-diisocyanate de diphénylméthane, éventuellement avec des proportions de 2,4'- diisocyanate de diphénylméthane et/ou de 2,2'-diisocyanate de diphénylméthane.

4. Composition de polyuréthane selon l'une quelconque des revendications 1 à 3, **caractérisée en ce que** le polyol contient au moins un triol.

5. Composition de polyuréthane selon l'une quelconque des revendications 1 à 4, **caractérisée en ce que** R¹ représente méthyle, éthyle, n-butyle ou benzyle.

6. Composition de polyuréthane selon l'une quelconque des revendications 1 à 5, **caractérisée en ce que** G représente furan-2-yle, 1,3-benzodioxol-5-yle, 1-naphtyle ou un radical de formule

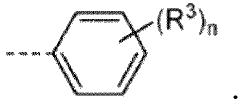

7. Composition de polyuréthane selon l'une quelconque des revendications 1 à 6, **caractérisée en ce que** G représente phényle, 2-méthylphényle, 3-méthylphényle, 4-méthylphényle, 4-isopropylphényle ou 4-méthoxyphényle.

8. Composition de polyuréthane selon l'une quelconque des revendications 1 à 7, **caractérisée en ce que** le rapport numérique entre des groupes oxazolidine et des groupes isocyanate se situe dans la plage de 0,2 à 0,45.

9. Composition de polyuréthane selon l'une quelconque des revendications 1 à 8, **caractérisée en ce que**, de plus, au moins une aldimine de formule 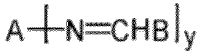 est présente, y représentant 2 ou 3, A représentant un radical organique comportant 2 à 25 atomes de C et B représentant un radical organique comportant 3 à 30 atomes de C.

10. Composition de polyuréthane selon la revendication 9, **caractérisée en ce que** le rapport numérique entre des groupes oxazolidine et des groupes isocyanate se situe dans la plage de 0,1 à 0,3 et le rapport numérique entre la somme des groupes oxazolidine et aldimine et des groupes isocyanate se situe dans la plage de 0,3 à 0,8.

11. Composition de polyuréthane selon l'une quelconque des revendications 1 à 10, **caractérisée en ce qu'**au moins un ingrédient supplémentaire est contenu, choisi dans le groupe constitué par des charges, des assouplissants, des oligomères de diisocyanate, des catalyseurs et des stabilisants.

12. Composition de polyuréthane selon l'une quelconque des revendications 1 à 11, **caractérisée en ce qu'**elle est à un composant et durcissant à l'humidité.

13. Procédé de collage et/ou d'étanchéification, comprenant les étapes
(i) d'application de la composition de polyuréthane selon l'une quelconque des revendications 1 à 12
- sur un premier substrat et mise en contact de la composition avec au moins un deuxième substrat à l'intérieur du temps de prise de la composition, ou
- sur un premier et sur un deuxième substrat et d'assemblage des deux substrats à l'intérieur du temps de prise de la composition, ou
- entre au moins deux substrats,
(ii) de durcissement de la composition par contact avec de l'humidité.

14. Procédé de revêtement et/ou d'étanchéification, comprenant les étapes
(i) d'application de la composition de polyuréthane selon l'une quelconque des revendications 1 à 12 sur au moins un substrat,
(ii) de durcissement de la composition par contact avec de l'humidité.

15. Article, obtenu par un procédé selon l'une quelconque des revendications 13 et 14.
